# EUROPEAN PATENT SPECIFICATION

(11) **EP 4 267 207 B1**
(45) Date of publication and mention of the grant of the patent: **21.10.2026**
(21) Application number: 21845008.8
(22) Date of filing: 22.12.2021
(51) Int. Cl.: A61L 9/12

(54) **SUBSTRATE FOR DIFFUSING VOLATILE SUBSTANCES**
SUBSTRAT ZUR DIFFUSION VON FLÜCHTIGEN SUBSTANZEN
SUBSTRAT POUR LA DIFFUSION DE SUBSTANCES VOLATILES

(30) Priority: 28.12.2020 ES 202031304
(43) Date of publication of application: 01.11.2023
(73) Proprietor: Zobele Holding SpA, 38100 Trento (IT)
(72) Inventor: LUQUE VERA, Sergio, 08019 BARCELONA (ES); SANCHO MARZO, Alberto, José, 08019 BARCELONA (ES); GARCÍA SUBIRATS, María, 08019 BARCELONA (ES); ALONSO CAÑADAS, David, 08019 BARCELONA (ES)
(74) Representative: Herrero & Asociados, S.L.
(86) International application number: PCT/EP2021/087433
(87) International publication number: WO 2022/144290

(56) References cited:
- WO-A2-2017/140860
- US-A- 4 144 318
- US-A1- 2010 136 128
- US-A1- 2013 213 309

## Description

The present invention relates to a substrate for diffusing volatile substances, which provides constant and long-lasting diffusion properties in air.

### Background of the invention

In air diffuser applications, the use of substrates containing a certain amount of volatile liquid substance and ensuring transport to an evaporation area is known.

The substrate must be able to hold a relatively large amount of liquid and therefore occupy a large volume. The common application requires a duration of at least 15 days of refill duration, which translates into storage volumes in the order of 1 to 10 ml.

In natural substrate materials, a high liquid storage capacity requires a large volume and thus a large surface area exposed to air. Since the evaporation rate is directly proportional to the evaporation area, a high volume also increases the evaporation rate and thus reduces the refill duration.

In addition, in solid substrate materials, the evaporation rate is proportional to the amount of liquid transported to the evaporation area (capillary properties), which change according to the amount of liquid contained in the substrate matrix. When the substrate is highly saturated (new refill), the transport and evaporation of liquid into the air is higher compared to low saturated conditions (end of life).

US4144318A discloses a mosquito coil composition comprising from 72-83% by weight based on dry ingredients of a carrier selected from sawdust having a particle size between 70 and 200 mesh, coconut shell flour, pyrethrum marc, and mixtures thereof, from 16-26% by weight based on dry ingredients potato starch and 0.5-3% by weight based on dry ingredients of an insecticide.

US2010136128A1 discloses a odor control fragrant additive comprises compacted pellets or granules infiltrated with fragrant oil. Cellulosic material, activated carbon and binder are mixed together and compacted to produce compacted pellets or granules. The cellulosic material preferably is peat moss, which swells up during absorption of fragrant oil and acts as fragrant oil storage. Fragrant oil is delivered to activated carbon that has large surface area, which evaporates the fragrance oil to release steady fragrance output.

US2013213309A1 discloses an animal litter, process for preparing the animal litter and a method for removal of animal waste using the animal litter are provided. The animal litter generally includes ground malt sprouts, a lubricant, and a cohesiveness agent. The animal litter is in the form of discrete plural particles which tend to agglomerate when wetted.

### Description of the invention

Therefore, there is a need for a substrate that offers both high storage capacity and a constant evaporation rate during its lifetime by low and constant capillarity properties.

Therefore, one purpose of the present invention is to provide a substrate that provides constant and durable air diffusion properties.

With the substrate according to the present invention it is possible to solve said drawbacks, providing other advantages which are described below.

The substrate for diffusing volatile substances according to the present invention it defined in claim 1 and it comprises a mixture of different natural ingredients with different granularities and a dispersible binder.

The substrate is made of natural particles and a dispersible binder material, such as flour-like materials, mixed with different granular grade materials, such as wood, e.g. sawdust. The composition is processed with water by conventional processes such as, for example, compression molding or extrusion. After processing, the substrate is dried to a solid state.

In addition, it may also comprise a preservative.

Advantageously, the percentages by weight in the dry state are as follows:
- dispersible binder: 15-45 %;
- natural material: 55-75 %; and
- preservative: 0,1-5 %.

More preferably, the percentages by weight in the dry state are as follows:
- dispersible binder: 20-30 %;
- natural material: 65-75 %; and
- preservative: 0,5-1,5 %.

According to a preferred embodiment, the granulometry of the dispersible binder is comprised between 100 and 250 µm, such as 180 µm.

The dispersible binder is flour and the natural material is wood, such as wood sawdust.

In addition, the preservative is preferably sodium benzoate.

The substrate provides a high storage capacity and a constant low capillarity, which ensures a long refill life at a constant evaporation rate.

The encapsulation of the substance within a granular matrix hinders the transport through the substrate to the evaporation area. This results in low capillary properties, which in turn also favors a lower gradient of substance between different areas of the substrate. Both effects result in a constant low supply of substance to the evaporation area and thus a constant and long-lasting evaporation rate.

### Description of a preferred embodiment

As an example, the substrate according to the present invention has the following composition.

| **Substrate composition** | **Granulometry (µm)** | |
|---|---|---|
| | **Interval** | **Preferential** |
| Dispersible binder (flour) | 100 - 250 | 180 |
| Natural material (wood) | 0-250 | <60 |
| Preservative | - | - |
| Water | - | - |

| **Substrate composition** | **% by weight, hydrated** | | **% by weight, dry** | |
|---|---|---|---|---|
| | **Interval** | **Preferential** | **Interval** | **Preferential** |
| Dispersible binder (flour) | 5-15 % | 7-10 % | 15-45 % | 20-30 % |
| Natural material (wood) | 15-30 % | 20-25 % | 55-75 % | 65-75 % |
| Preservative | 0.1-5 % | 0.5-1 % | 0.1-5 % | 0.5-1.5 % |
| Water | 40-80 % | 50-70 % | - | - |

| **Substrate composition** | **Grain size range (µm)** | | **% by weight, hydrated** | | **% by weight, dry** | |
|---|---|---|---|---|---|---|
| | **Interval** | **Preferential** | **Interval** | **Preferential** | **Interval** | **Preferential** |
| Dispersible binder (flour) | 100-250 | 180 | 5-15 % | 7-10 % | 15-45 % | 20-30 % |
| Natural material (wood) | 0-60 | <60 | 15-30 % | 20-25 % | 55-75 % | 65-75 % |
| Preservative | - | - | 0.1-5 % | 0.5-1 % | 0.1-5 % | 0.5-1.5 % |
| Water | - | - | 40-80 % | 50-70 % | - | - |

Furthermore, according to a preferred embodiment, the substrate according to the present invention is disc-shaped, although it may have a different shape, and its thickness changes from 0.1 to 20 mm.

A non-limiting example of substrate composition is:
Dispersible binder (flour): 25.8 % by weight on dry condition
Natural material (wood): 73.2 % by weight in dry condition
Preservative (sodium benzoate): 1 % by weight on dry condition

Although reference has been made to a specific embodiment of the invention, it is obvious to a person skilled in the art that the substrate described herein is susceptible to numerous variations and modifications, and that all the details mentioned may be replaced by technically equivalent ones without departing from the scope of protection defined by the appended claims.

## Claims

1. A substrate for diffusing volatile substances, the substrate comprising:
- a dispersible binder that is flour; and
- a natural material that is wood,
**characterized in that** the granulometry of the wood is less than 60 µm.

2. The substrate for diffusing volatile substances according to claim 1, wherein the percentages by weight in the dry state are as follows:
- dispersible binder: 15-45 %; and
- natural material: 55-75 %.

3. The substrate for diffusing volatile substances according to claim 2, wherein the percentages by weight in the dry state are as follows:
- dispersible binder: 20-30 %; and
- natural material: 65-75 %.

4. The substrate for diffusing volatile substances according to claims 1 or 3, further comprising a preservative.

5. The substrate for diffusing volatile substances according to claim 4, wherein the percentage by weight in dry state of the preservative is between 0.1 and 5 %, in particular between 0.5 and 1.5 %.

6. The substrate for diffusing volatile substances according to claim 1, wherein the natural material is wood sawdust.

7. The substrate for diffusing volatile substances according to claim 4 or 5, wherein the preservative is sodium benzoate.

## Patentansprüche

1. Substrat zur Diffusion von flüchtigen Substanzen, wobei das Substrat umfasst:
- ein dispergierbares Bindemittel, das Mehl ist; und
- ein natürliches Material, das Holz ist,
**dadurch gekennzeichnet, dass** die Korngröße des Holzes weniger als 60 µm beträgt.

2. Substrat zur Diffusion von flüchtigen Substanzen nach Anspruch 1, wobei die Gewichtsprozente im trockenen Zustand wie folgt sind:
- dispergierbares Bindemittel: 15-45 %; und
- natürliches Material: 55-75 %.

3. Substrat zur Diffusion von flüchtigen Substanzen nach Anspruch 2, wobei die Gewichtsprozente im trockenen Zustand wie folgt sind:
- dispergierbares Bindemittel: 20-30 %; und
- natürliches Material: 65-75 %.

4. Substrat zur Diffusion von flüchtigen Substanzen nach Anspruch 1 oder 3, ferner umfassend ein Konservierungsmittel.

5. Substrat zur Diffusion von flüchtigen Substanzen nach Anspruch 4, wobei der Gewichtsprozentsatz des Konservierungsmittels im trockenen Zustand zwischen 0,1 und 5 % beträgt, insbesondere zwischen 0,5 und 1,5 %.

6. Substrat zur Diffusion von flüchtigen Substanzen nach Anspruch 1, wobei das natürliche Material Holzsägemehl ist.

7. Substrat zur Diffusion von flüchtigen Substanzen nach Anspruch 4 oder 5, wobei das Konservierungsmittel Natriumbenzoat ist.

## Revendications

1. - Substrat pour diffuser des substances volatiles, le substrat comprenant :
- un liant dispersible qui est la farine ; et
- un matériau naturel qui est le bois,
**caractérisé en ce que** la granulométrie du bois est inférieure à 60 µm.

2. - Substrat pour diffuser des substances volatiles selon la revendication 1, dans lequel les pourcentages en poids à l'état sec sont les suivants :
- liant dispersible : de 15 à 45 % ; et
- matériau naturel : de 55 à 75 %.

3. - Substrat pour diffuser des substances volatiles selon la revendication 2, dans lequel les pourcentages en poids à l'état sec sont les suivants :
- liant dispersible : de 20 à 30 % ; et
- matériau naturel : de 65 à 75 %.

4. - Substrat pour diffuser des substances volatiles selon la revendication 1 ou 3, comprenant en outre un conservateur.

5. - Substrat pour diffuser des substances volatiles selon la revendication 4, dans lequel le pourcentage en poids à l'état sec du conservateur est compris entre 0,1 et 5 %, en particulier entre 0,5 et 1,5 %.

6. - Substrat pour diffuser des substances volatiles selon la revendication 1, dans lequel le matériau naturel est la sciure de bois.

7. - Substrat pour diffuser des substances volatiles selon la revendication 4 ou 5, dans lequel le conservateur est le benzoate de sodium.
